# EUROPEAN PATENT SPECIFICATION

(11) **EP 1 743 588 B1**
(45) Date of publication and mention of the grant of the patent: **11.03.2009**
(21) Application number: 06253712.1
(22) Date of filing: 14.07.2006
(51) Int. Cl.: A61B 18/14

(54) **Electrosurgical electrode with silver**
Elektrochirurgische Elektrode mit Silber
Électrode electrochirurgicale avec argent

(30) Priority: 14.07.2005 US 180809
(43) Date of publication of application: 17.01.2007
(73) Proprietor: Garito, Jon C., Oceanside, New York 11572 (US); Ellman, Alan G., Oceanside, New York 11572 (US)
(72) Inventor: Garito, Jon C., Oceanside, New York 11572 (US); Ellman, Alan G., Oceanside, New York 11572 (US)
(74) Representative: Cloughley, Peter Andrew

(56) References cited:
- WO-A-98/16162
- US-A- 5 713 942
- US-A- 5 925 039
- US-A1- 2004 236 203
- US-B1- 6 540 745
- US-B1- 6 673 072
- PATENT ABSTRACTS OF JAPAN vol. 013, no. 169 (C-587), 21 April 1989 (1989-04-21) & JP 63 317073 A (JAPAN SPECTROSCOPIC CO; others: 01), 26 December 1988 (1988-12-26)

## Description

This invention relates to electrosurgery, and in particular to an electrode useful in various electrosurgical procedures.

### BACKGROUND OF THE INVENTION

Electrosurgery is a common procedure for dentists, doctors, and veterinarians. Electrosurgical handpieces are commercially available that will accommodate a wide variety of electrodes shapes and sizes, such as needles, blades, scalpels, balls and wire loops. Also, multi-function electrodes are available.

An electrosurgical handpiece for blades is described in USP 4,754,754. This is an instrument that can be connected to a source of electrosurgical currents and that provides a slitted collet for receiving the shank of a standard disposable scalpel blade. The instrument can be used in many surgical procedures in which a conventional scalpel is employed, mainly for general cutting procedures. It has the advantage of providing electrosurgical currents at the sharp edge of the scalpel which assist in cutting tissue while at the same time providing a coagulation effect. Other known electrode shapes include a curet, as described in our patent No. 5,913,864. This is a circular band with one sharpened edge for use in an electrosurgical dermatological curretage procedure. Another shape is the well-known ball electrode which is a spherical ball on the end of an electrode shank which is used for coagulation. Still another shape is a flat round disc with a tapered edge useful for vaporizing lesions and tumor tissue, as described in our issued patent No. 6,610,057. See also our patent No. 6,673,072 relating to an electrode with a projecting point.

While these various shaped electrodes are suitable for their intended purposes, occasions arise from time-to-time when the electrode during use may tend to stick to the cut or coagulated tissue, which can prove undesirable. Similarly, during use, the electrode may overheat, which can lead to undesirable tissue damage.

Another problem may arise when the electrode is used to slice a thin tissue specimen for biopsy purposes. In this application, the physician performs histological studies by examining the specimen surface under a microscope, for example, searching for potentially tumor-forming cells. It is desirable that the surface represent as closely as possible the underlying tissue, meaning the surface cells at the severed surface should exhibit as little alteration and damage as possible. For example, an ordinary cold steel scalpel used to slice off a specimen typically forms at the severed surface damage extending to a certain depth. Using a typical elcctrosurgically-activated electrode, such as a scalpel or needle, loop or blade of tungsten or steel provides an improvement in that the damage extends to a lesser depth.

### SUMMARY OF THE INVENTION

An object of the invention is an improved electrosurgical electrode capable of performing cutting or coagulation with an active edge or point.

Another object of the invention is an electrosurgical electrode that may tend to stick to tissue less than other known electrodes.

Still another object of the invention is an electrosurgical electrode capable of cutting through tissue and causing less alteration and damage to the cut tissue surfaces.

According to one aspect of the invention, there is provided an electrosurgical electrode comprising: a) a body comprising a core metal consisting essentially of molybdenum, b) the body having a first end configured for attaching or mounting to an electrosurgical handpiece, c) the body having a second active end opposite to the first end capable of supplying electrosurgical currents to tissue when the first end is connected to electrosurgical apparatus, d) said active second end comprising an active surface that is configured to perform a cutting or coagulation action when activated with electrosurgical currents and the active surface brought into contact with the tissue, characterized by e) a cladding metal tightly bonded and adherent to the core metal and consisting essentially of silver with 1.5-4% of germanium and 1-2% of indium.

According to another aspect of the invention, there is provided an electrosurgical electrode comprising: a) a body, b) the body having a shank and a first end configured for attaching or mounting to an electrosurgical handpiece, c) the body having a second active end opposite to the first end capable of supplying electrosurgical currents to tissue when the first end is connected to electrosurgical apparatus, d) wherein either: (i) said body is generally ball-shaped and said active second end comprises an active surface that is configured to perform a cutting or coagulation action when activated with electrosurgical currents and the active surface brought into contact with the tissue, or (ii) said body is generally needle-shaped and said active second end comprises a sharpened point that is configured to perform a cutting action when activated with electrosurgical currents and brought into contact with the tissue, characterized in that e) the body comprises a metal consisting essentially of silver with 1.5-4% of germanium and 1-2% of indium.

According to yet another aspect of the invention, there is provided a method for manufacturing an electrosurgical electrode as set forth above, comprising fabricating the body by wrapping a relatively thick foil of the cladding metal about a relatively thick wire or rod of the core metal, heating at an elevated temperature below the melting or softening point of the cladding metal and the molybdenum to tightly bond the cladding metal to the wire or rod core, drawing the relatively thick clad wire or rod through a first die which reduces the diameter by about 10-20%, then annealing the drawn clad wire or rod to restore the molybdenum ductility, then drawing the thinner clad wire or rod through a second die which reduces the diameter by about another 10-20%, again annealing to restore the molybdenum ductility, and so on until the resultant clad wire or rod has reached the smaller diameter of 0.10-0.64 mm (4-25 mils).

It has been suggested in the past to use noble metals as electrode materials because of their reduced electrical resistance that therefore allows the use of lower electrosurgical currents which in turn means that less heat is developed at the incision. It has also been suggested that an electrosurgical electrode should have an outer surface of a noble metal because the noble metal is bio-compatible with tissue and also reduces the tendency for the

electrode to stick to the tissue. Thus the prior art has suggested the use of pure silver or gold and silver coated core metals such as copper, brass, or stainless steel. See for example, US 2004/0236203 A1 which describes a special silver alloy capable of generating far infra-red radiation. We have tried to make and use such prior art electrodes but have not been fully satisfied with the results, for various reasons.

The principal feature of our invention is an electrosurgical electrode with a core mainly of a refractory, reasonably ductile metal and clad with a silver alloy with a small percentage of other constituents. Preferably the core is molybdenum. Preferably the cladding is about 93-98% by weight silver with about 1.5-4% by weight of germanium and 1-2% by weight of indium. A preferred composition is a cladding of 97% silver with 2% germanium and 1% indium, on a molybdenum core. A small percentage of copper may also be present in the core or cladding.

"Cladding" as used herein is defined to mean a process that embeds a coating material into the core. It is not an ordinary coating, such as that obtained by plating or electro-plating or by vapor-depositing a coating material onto the core. The cladding process is much more intense and actually causes the cladding material and core material to interdiffuse and alloy at their interface over a significant depth. This produces an extremely strong bond between the cladding material and the core. This is critical to the invention. The reason is that, for a typical needle or loop electrode, the smaller the diameter of the needle or of the loop, the less likely that the damage at the incision will be deep. Preferably, the needle electrode of the invention employing the silver-alloy clad core metal has an overall diameter between about 0.004 and 0.025 inches, preferably, between about 0.006 and 0.020 inches (6-20 mils, where 0.001 inch = 1 mil) with a pointed end. Manufacturing such a shaped electrode, which also applies to loop electrodes, typically requires that a coated core wire or rod be drawn down from a starting diameter of say about 450 mils to the final size desired. The drawing process could have a tendency to cause damage to the coating such as by chipping or cracking or stripping off ordinary coatings. We have found that the cladding process that is preferred, in contrast, produces such a strong bond between the coating and the core that the larger diameter needle is easily drawn, as explained below, down to the required very fine size without causing damage to the outside silver-alloy coating.

For making fine needles or wire for use as a loop, it is difficult to start with a thin enough clad wire such that only a single drawing step suffices to produce the desired fine needle or wire. On the other hand, if one starts with a thicker clad wire, then it is difficult if not impossible for a single drawing step to produce the desired fine needle or wire. Hence, several successive drawing steps are required. But, each drawing step even with ductile metals typically work-hardens the clad wire making it difficult to carry out a succeeding drawing step on the resultant work-hardened wire. Hence, it is preferred that each drawing step be followed by an annealing step which allows the drawn wire to regain its ductile properties in order to ease the next drawing step until the desired fine needle or wire is obtained. Thus, in the preferred cladding process, the starting relatively thick clad wire or rod is drawn through a first die which reduces the diameter by say 10-20%, annealed to restore the molybdenum ductility, drawn through a second die which reduces the diameter by another 10-20%, again annealed to restore the molybdenum ductility, and so on until the resultant wire has reached the preferred small diameter desired.

The thickness of the cladding is typically about 5-15% of the overall needle diameter. The mainly high melting point molybdenum core provides a stiff electrode body. The silver-alloy cladding provides the low resistance wanted to reduce heating of the tissue during use. We have also found that the silver-alloy cladding is bio-compatible with the tissue and no undesirable side-effects arise from contact between the silver-alloy and tissue.

While it is preferred that the silver-alloy cladding be thin, preferably about 1 mil thick, forming a sharpened point at the end for a needle electrode may have a tendency to remove the cladding at the tapered end. In such cases, it is preferred that a thicker cladding be used, such as 2-3 mils. In accordance with another feature of the invention, where a needle electrode with a very sharp pointed end is desired, then it is preferred that the entire electrode be composed of the silver alloy, i.e., with about 1.5-4% by weight of germanium and 1-2% by weight of indium, remainder silver. In this case, it is preferred that the starting stock be a straight silver alloy rod with a diameter of about 30-40 mils, preferably about 35 mils. Then it is possible to grind the working end down to a sharp point while retaining sufficient strength in the supporting rod for use as a needle electrode.

The same silver-alloy clad molybdenum core material can also be used to make blade, scalpel, and curet shaped electrodes, but the process has not yet been adequately refined for the manufacture of the typical ball-shaped electrode. In the latter case, it is preferred that the silver-alloy composition alone be used, without the molybdenum core, but the electrode preferably is specially shaped to enhance its performance. Preferably, the active part of the ball is not spherical, but has a truncated conical shape, with the narrower or distal end at the active tip. Preferably, this specially-shaped electrode is connected at its proximal end to a brass rod to serve as the electrode shank. Proximal and distal are taken with respect to the shank end of the electrode which is held by the electrosurgical handpiece.

We have found that best results are obtained when the electrosurgical electrode in accordance with the invention is used with an electrosurgical instrument capable of generating radio-frequency electrosurgical currents in the 2-4 MHz range. Examination of the severed surfaces of tissue cut with the loop electrode of the invention connected to an electrosurgical instrument generating 4 MHz cutting electrosurgical currents has a damaged tissue depth less than one-half of that cut with a cold scalpel, and at least 20% less than that cut with the same needle but using electrosurgical currents at KHz frequencies. Electrosurgical instruments capable of generating radio frequency cutting currents in the MHz range are available from Ellman International, Inc. of Oceanside, New York. See also our patents Nos. 5,954,686 and 6,238,388 relating to MHz frequency electrosurgical units.

The cladding processes that are preferably used to provide the tightly-bonded alloy coating in accordance with the invention may be briefly described in the following example.

A foil of silver-alloy that was approximately 0.010" thick and 3" x 3" square was wrapped and then bonded by prolonged heating at an elevated temperature below the melting or softening point of the silver alloy around a molybdenum rod that was around 3/8ths of an inch in diameter. The resultant clad rod was drawn through a series of drawing dies each with a smaller aperture gradually reducing the diameter of the clad rod. Preferably, at the end of each drawing step, the wire was put into a furnace and heated at an elevated temperature to anneal and soften the material up from the resultant cold working it received during the drawing reduction process. These steps are repeated until the wire is at the desired size. The end result is a wire with a core of molybdenum and an outer clad layer of the silver-alloy. The cross-section would look similar to a pencil.

For a 9 mil electrode, the layer of the silver alloy is about .001" thick on about a 7 mil core. For a 20 mil needle electrode, alloy again was .001" thick and the core about 18 mils in diameter. For grinding the end to a fine point, a thicker alloy layer should be used.

As for the ball electrodes, these are prepared by turning from a solid rod stock of the silver-alloy alone. No cladding is necessary here for strength. The shaped ball is then assembled to a brass tube to serve as the shank. Small diameter ball shapes can have an overall diameter of about 5.0mm. With the preferred geometry having the narrow cone end preferably has a diameter of about 1.0mm at the narrow end.

For the silver-alloy clad core metal, the cladding process described ensures that sufficient mutual diffusion occurs such that the cladding interface is essentially embedded in the core metal. This produces the tightness of bonding required for the wire to withstand the subsequent drawing down process without cracking or chipping. Since the final needle product during use does not undergo any significant physical wear and tear, being used only as an electrosurgical electrode where the pressure against the tissue as applied by the surgeon is usually light, wearing of the coating is minimal and thus thin coatings are adequate for the purpose.

While an ultra-fine needle or a fine loop mainly of molybdenum clad with the silver-alloy is preferred as it produces the most benefits in terms of reducing tissue damage at the incision and reducing generated heat, especially for preparing biopsy specimens, the invention can also be used with, for example, blade electrodes or scalpel electrodes or ball or loop electrodes, and can also be used with forceps electrodes. This applies to both unipolar and bipolar electrodes. In the case of the forceps, the reduced heat generated by the electrosurgical currents will reduce the tendency of the forceps tips to stick to tissue.

Studies have been made comparing the surface alteration of the tissue when an incision (removal of a slice) is made by a tungsten loop electrode and a loop electrode in accordance with the invention comprising a core of molybdenum clad with an alloy of 97% silver with 2% germanium and 1% indium. The studies all used an electrosurgical instrument supplied by the Ellman company and all used the 4 MHz instrument setting. The specimens were Facial Nevus shavings (5 micron slice) separately made with a tungsten loop and the silver alloy loop of the invention. Of the six biopsy specimens taken, the measurements of the three specimens taken with the tungsten loop indicated a depth of thermal damage as high as 30 microns, whereas the measurements of the comparable three specimens taken with the silver alloy loop of the invention indicated a depth of thermal damage in micrometers no greater than 10 microns. Also, the silver alloy specimens were cut using the fully filtered waveform at the 12 watt setting, resulting in less heating. So it is clear that the silver alloy electrode of the invention offers on average less surface damage and thus inevitably less pain and suffering and faster healing at the biopsy site. In general, all three of the typical waveforms are usable with advantage, including the fully filtered, fully rectified and partially rectified, to do excisions, incisions, and coagulation. There will usually be a preferred power setting and waveform for the various procedures, to avoid arcing and undue tissue damage. With the silver alloy of the invention, we have found that typically, a power setting and waveform can be chosen by the surgeon that well matches the use of the silver alloy electrode and minimizes heating and undue damage to the tissue. While the 4 MHz frequency is preferred, advantageous results will be obtained with lower frequencies also.

The foregoing clearly demonstrates the superiority of the electrode in accordance with the invention for this application, and it can reasonably be forecast from these studies and others that an improvement will result in most electrosurgical procedures, because less tissue damage where the incision is made usually results in less pain and trauma for the patient and accelerates the healing process.

The various features of novelty which characterize the invention are pointed out with particularity in the claims annexed to and forming a part of this disclosure. For a better understanding of the invention, its operating advantages and specific objects attained by its use, reference should be had to the accompanying drawings and descriptive matter in which there are illustrated and described the preferred embodiments of the invention, like reference numerals or letters signifying the same or similar components..

### BRIEF DESCRIPTION OF THE DRAWINGS

In the drawings:
Fig. 1 is a plan view of one form of unipolar needle electrosurgical electrode according to the invention;
Fig. 2 is a perspective view of another form of bipolar forceps electrosurgical electrode according to the invention;
Fig. 3 is a perspective view of the working end of a unipolar scalpel electrode of the invention;
Fig. 4 is a perspective view of a less-preferred ball electrode of the invention;
Fig. 5 is a top view of yet another form of electrosurgical electrode according to the invention, in this case, a loop, shown attached to a schematic of the handpiece described in the >754 patent which is in turn electrically connected to electrosurgical apparatus. What is not shown here and in the other drawings is an electrically-insulating coating that covers the back end of the electrode as described in the patent;
Fig. 6 is a perspective view of a bipolar electrode in accordance with the invention;
Fig. 7 is a perspective view of a preferred ball electrode in accordance with the invention;
Figs. 8 and 9 are, respectively, a schematic of a longitudinal and horizontal cross-section of one form of a needle electrode in accordance with the invention.

### DETAILED DESCRIPTION OF THE PREFERRED EMBODIMENTS

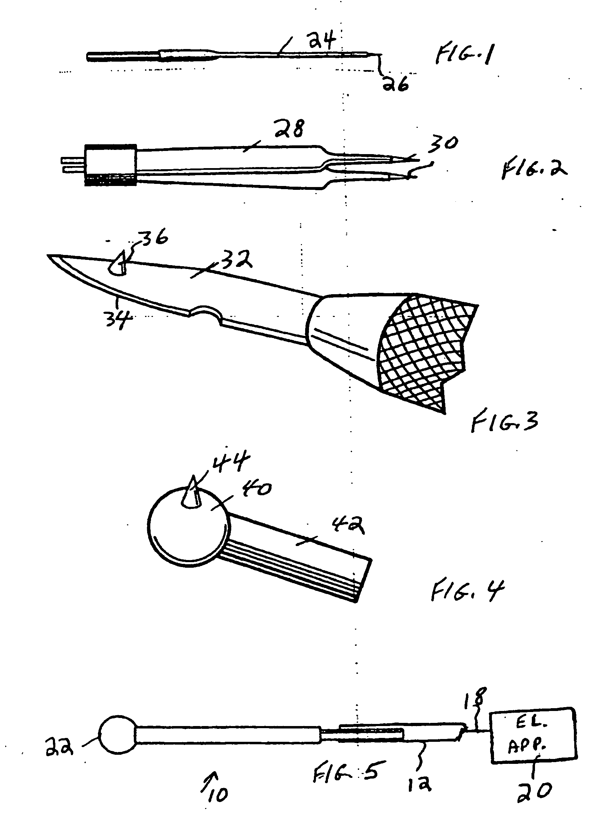

Fig. 5 is a plan view of a unipolar electrosurgical electrode 10 according to the invention attached to the nosepiece 12 of the hollow handpiece described in the >754 patent. The latter comprises at its end a cable 18 connected at its opposite end to a connector (not shown) for plugging into a standard electrosurgical apparatus 20 supplying electrosurgical currents to the electrode 10 having a working end 22 in the form of a loop. In this instance, the loop would be constituted of the silver-alloy clad molybdenum of the invention.

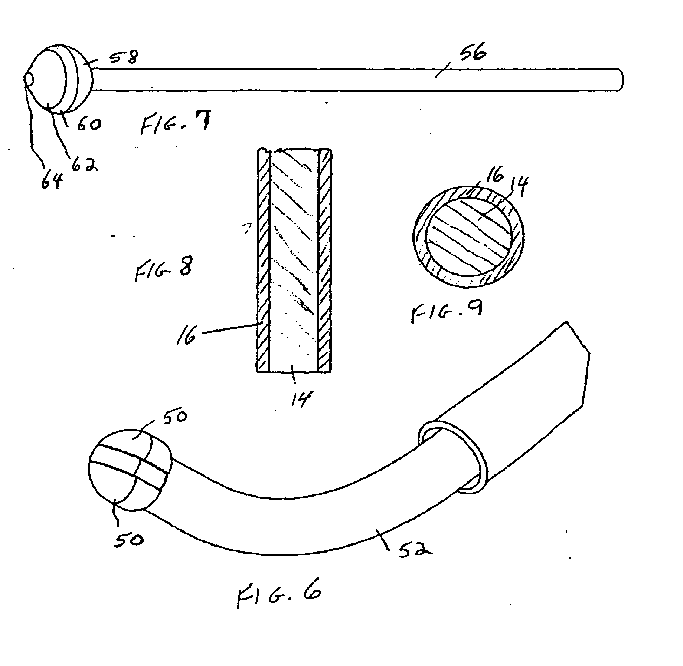

In the variation illustrated in Fig. 1, the electrode is a needle electrode 24, preferably used for the cutting of biopsy specimens. In this instance, the pointed needle end 26 would be constituted of the silver-alloy clad molybdenum of the invention, and would be operated as a unipolar electrode. Figs. 8 and 9 are, respectively, schematics of a longitudinal and horizontal cross-section of the needle electrode of Fig. 1. The core is designated 14, and the cladding 16. The drawings are not to scale.

As described above, another preferred embodiment of the invention is a silver alloy needle electrode with a sharpened end, in this case without the molybdenum core. The electrode can have a straight shank but is preferably of a larger diameter than when the silver alloy is a cladding on a molybdenum core.

Fig. 2 illustrates a conventional bipolar forceps 28, which in this instance, the sharpened tips 30 would be constituted of the silver-alloy clad molybdenum of the invention. Forceps can also be operated as a unipolar electrode.

Fig. 3 illustrates a scalpel blade 32 as described in the '754 patent with a sharpened edge surface 34. In accordance with the present invention, the sharpened edge 34 would be constituted of the silver-alloy clad molybdenum of the invention. The blade may be optionally provided with a short conical point 36 provided near but back of the sharpened edge 34 and on the flat surface. The point 36 preferably projects approximately orthogonally upward, and if present and not too sharp may be constituted of the silver-alloy clad molybdenum of the invention.

Fig. 4 illustrates a typical ball unipolar electrode 40 mounted on the end of a shaft 42. This is a less preferred version of the ball-shaped electrode as it does not perform as well as the preferred truncated ball shape of Fig. 7. In accordance with the present invention, the ball surface or a short conical point 44 if optionally present provided on the side of the ball back of the front surface would be constituted alone of the silver-alloy of the invention.

Fig. 6 illustrates a typical dual ball bipolar electrode 50 mounted on the end of a retractable and extendable shaft 52 as described in USP 6,231,571. The ball ends 50 would be preferably formed of the silver alloy of the invention. Note that the active ball ends 50 are flattened and not spherical.

The preferred ball shape is illustrated in Fig. 7. The ball end of the silver alloy (no cladding in this case) is bonded in any suitable manner as by brazing to a brass rod or tube 56 that serves as the electrode shank. The back half 58 is spherical. It is then optionally followed by a short cylindrical shape 60, and then by a tapered conical shape 62 down to the distal end 64 which is flattened. The latter can be for example about 1mm in diameter for a 5mm ball. The flattened end 64 serves to concentrate the electrosurgical currents and thus requires less power and results in less heating of the electrode.

It is clear that the invention is not limited to these illustrative embodiments and includes within its scope other shapes and styles of known electrodes, both unipolar and bipolar, especially those with sharpened points or edges or narrow edges such as a loop.

The electrosurgical apparatus 20 preferably is an ultra high frequency (RF) radiosurgical energy source, which operates in the range of about 2-4 MHz, preferably 3.8-4.0 MHz. Studies have shown that the 3.8-4.0 MHz frequency range is the preferred RF energy to incise and coagulate tissue because tissue thermal necrosis is minimal and, when interfaced with the electrosurgical electrode of the invention, provides excellent cutting and hemostasis especially for removal of cancerous tissue and also for cutting specimens for biopsy studies. An example of suitable electrosurgical apparatus is the Model SURGITRON Dual-Frequency electrosurgical unit manufactured by and available from Ellman International, Inc. of Oceanside, New York.

What is not shown in the drawings are the presence of electrically-insulating coatings on the conductive parts of the electrode that support the active end and that are not involved in the surgical procedure for preventing inadvertent bums to the patient.

In the operation of the embodiments of the invention, activation of the electrosurgical unit 20 causes the flow of electrosurgical currents from the electrode working end when applied against or close to the tissue to be destroyed. With the electrodes of Figs.l, 3 and 5, typically the sharpened points or edge surfaces 26, 30, 34, 36 22, respectively, is used to perform a cutting operation on the tissue to be treated. Controlled vaporization and evaporation of, for example, tumor tissue can be achieved especially with the 4 MHz radiofrequency apparatus. The cutting current, fully rectified waveform is used. Once the RF is applied, the knife edge blade is moved across the skin until the desired amount of tissue is vaporized. By raising the power (wattage) of the 4 MHz radiosurgery unit the greater the cutting ability and the amount of tissue destruction and vaporization over a unit period of time. The ball electrodes are often used for coagulation purposes as well as the forceps.

The electrodes of the invention can be made in a sterile disposable single use design but it is not limited to single use. It can also be made in reusable autoclaveable material.

Other variations in the shape of the electrosurgical electrode working end will provide the same or similar benefits and advantages as will be evident to those skilled in the art.

While the invention has been described in connection with preferred embodiments, it will be understood that modifications thereof within the principles outlined above will be evident to those skilled in the art and thus the invention is not limited to the preferred embodiments but is intended to encompass such modifications.

## Claims

1. An electrosurgical electrode (24) comprising:
a) a body comprising a core metal (14) consisting essentially of molybdenum,
b) the body having a first end configured for attaching or mounting to an electrosurgical handpiece,
c) the body having a second active end (26) opposite to the first end capable of supplying electrosurgical currents to tissue when the first end is connected to electrosurgical apparatus (20),
d) said active second end (26) comprising an active surface that is configured to perform a cutting or coagulation action when activated with electrosurgical currents and the active surface brought into contact with the tissue, **characterized by**
e) a cladding metal (16) tightly bonded and adherent to the core metal and consisting essentially of silver with 1.5-4% of germanium and 1-2% of indium.

2. An electrosurgical electrode (24) as set forth in claim 1, wherein the active surface is a point.

3. An electrosurgical electrode (24) as set forth in claim 2, wherein the electrode body comprises a needle having a shaft and a pointed end (26), the shaft having a diameter of about 0.10-0.64 mm (4-25 mils).

4. An electrosurgical electrode (24) as set forth in claim 3, wherein the cladding metal (16) has a thickness of about 0.025-0.18 mm (1-7 mils).

5. An electrosurgical electrode (24) as set forth in claim 4, wherein the cladding (16) thickness is about 5-15% of the overall thickness of the electrode (24).

6. An electrosurgical electrode (32) as set forth in claim 1, wherein the electrode (32) comprises a generally flat part having along a front or side portion of its periphery, projecting sideways or forwardly of the flat part in a direction away from the first end, an exposed sharpened edge (34) serving as the active surface.

7. An electrosurgical electrode (32) as set forth in claim 6, further comprising a projecting point (36) projecting orthogonally to the flat part of the electrode (32).

8. An electrosurgical electrode (24) as set forth in claim 1, wherein the electrode (10) comprises a loop (22).

9. An electrosurgical electrode (24) as set forth in claim 1, wherein the electrode (10) comprises a forceps (28).

10. An electrosurgical electrode (40) comprising:
a) a body,
b) the body having a shank (42) and a first end configured for attaching or mounting to an electrosurgical handpiece,
c) the body having a second active end opposite to the first end capable of supplying electrosurgical currents to tissue when the first end is connected to electrosurgical apparatus,
d) wherein either:
(i) said body is generally ball-shaped and said active second end comprises an active surface that is configured to perform a cutting or coagulation action when activated with electrosurgical currents and the active surface brought into contact with the tissue, or
(ii) said body is generally needle-shaped and said active second end comprises a sharpened point that is configured to perform a cutting action when activated with electrosurgical currents and brought into contact with the tissue,
**characterized in that**
e) the body comprises a metal consisting essentially of silver with 1.5-4% of germanium and 1-2% of indium.

11. An electrosurgical electrode (40) as set forth in claim 10, wherein the active surface is configured with a truncated conical shape (62) terminating in a generally flattened distal end (64).

12. An electrosurgical electrode (40) as set forth in claim 10, wherein the ball-shaped body comprises a sharpened point (44).

13. An electrosurgical electrode (24) as set forth in claim 1, in combination and for use with electrosurgical apparatus (20) capable of supplying RF electrosurgical currents at a frequency of about 3.8-4 MHz.

14. An electrosurgical electrode (24) as set forth in claim 1, in combination and for use with electrosurgical apparatus (20) capable of supplying electrosurgical currents in the 2-4 MHz range.

15. A method for manufacturing an electrosurgical electrode (24) as set forth in claim 1, comprising fabricating the body by wrapping a relatively thick foil of the cladding metal (16) about a relatively thick wire or rod of the core metal (14), heating at an elevated temperature below the melting or softening point of the cladding metal (16) and the molybdenum to tightly bond the cladding metal (16) to the wire or rod core (14), drawing the relatively thick clad wire or rod through a first die which reduces the diameter by about 10-20%, then annealing the drawn clad wire or rod to restore the molybdenum ductility, then drawing the thinner clad wire or rod through a second die which reduces the diameter by about another 10-20%, again annealing to restore the molybdenum ductility, and so on until the resultant clad wire or rod has reached the smaller diameter of 0.10-0.64 mm (4-25 mils).

## Patentansprüche

1. Elektrochirurgische Elektrode (24), die Folgendes umfasst:
a) einen Körper, der ein Kernmetall (14) umfasst, das im Wesentlichen aus Molybdän besteht,
b) wobei der Körper ein erstes Ende hat, das dafür konfiguriert ist, an einem elektrochirurgischen Handstück befestigt oder angebracht zu werden,
c) wobei der Körper ein zweites, aktives, Ende (26) hat, entgegengesetzt zu dem ersten Ende und dazu in der Lage, einem Gewebe elektrochirurgische Ströme zuzuführen, wenn das erste Ende mit einer elektrochirurgischen vorrichtung (20) verbunden ist,
d) wobei das zweite, aktive, Ende (26) eine aktive Fläche umfasst, die dafür konfiguriert ist, einen Schneid- oder Koagulationsvorgang auszuführen, wenn sie mit elektrochirurgischen Strömen aktiviert wird und die aktive Fläche in Berührung mit dem Gewebe gebracht wird, **gekennzeichnet durch**
e) ein Plattierungsmetall (16), das eng an das Kernmetall gebunden ist und daran haftet und im Wesentlichen aus Silber mit 1,5 bis 4 % Germanium und 1 bis 2 % Indium besteht.

2. Elektrochirurgische Elektrode (24) nach Anspruch 1, wobei die aktive Fläche eine Spitze ist.

3. Elektrochirurgische Elektrode (24) nach Anspruch 2, wobei der Elektrodenkörper eine Nadel umfasst, die einen Schaft und ein zugespitztes Ende (26) hat, wobei der Schaft einen Durchmesser von etwa 0,10 bis 0,64 mm (4 bis 25 Milli-Inch) hat.

4. Elektrochirurgische Elektrode (24) nach Anspruch 3, wobei das Plattierungsmetall (16) eine Dicke von etwa 0,025 bis 0,18 mm (1 bis 7 Milli-Inch) hat.

5. Elektrochirurgische Elektrode (24) nach Anspruch 4, wobei die Plattierungsdicke etwa 5 bis 15 % der Gesamtdicke der Elektrode (24) beträgt.

6. Elektrochirurgische Elektrode (32) nach Anspruch 1, wobei die Elektrode (32) einen im Allgemeinen flachen Teil umfasst, der längs eines vorderen oder seitlichen Abschnitts ihres Umfangs, von dem flachen Teil seitlich oder nach vorn vorspringend in einer Richtung, weg von dem ersten Ende, eine freigelegte geschärfte Kante (34) hat, die als die aktive Fläche dient.

7. Elektrochirurgische Elektrode (32) nach Anspruch 6, die ferner eine vorspringende Spitze (36) umfasst, die senkrecht zu dem flachen Teil der Elektrode (32) vorspringt.

8. Elektrochirurgische Elektrode (24) nach Anspruch 1, wobei die Elektrode (10) eine Schleife (22) umfasst.

9. Elektrochirurgische Elektrode (24) nach Anspruch 1, wobei die Elektrode (10) eine Pinzette (28) umfasst.

10. Elektrochirurgische Elektrode (40), die Folgendes umfasst:
a) einen Körper,
b) wobei der Körper einen Schaft (42) und ein erstes Ende hat, das dafür konfiguriert ist, an einem elektrochirurgischen Handstück befestigt oder angebracht zu werden,
c) wobei der Körper entgegengesetzt zu dem ersten Ende ein zweites, aktives, Ende hat, das dazu in der Lage ist, einem Gewebe elektrochirurgische Ströme zuzuführen, wenn das erste Ende mit einer elektrochirurgischen Vorrichtung verbunden ist,
d) wobei entweder
(i) der Körper im Allgemeinen kugelförmig ist und das zweite, aktive, Ende eine aktive Fläche umfasst, die dafür konfiguriert ist, einen Schneid- oder Koagulationsvorgang auszuführen, wenn sie mit elektrochirurgischen Strömen aktiviert wird und die aktive Fläche in Berührung mit dem Gewebe gebracht wird, oder
(ii) der Körper im Allgemeinen nadelförmig ist und das zweite, aktive, Ende eine geschärfte Spitze umfasst, die dafür konfiguriert ist, einen Schneidvorgang auszuführen, wenn sie mit elektrochirurgischen Strömen aktiviert wird und die aktive Fläche in Berührung mit dem Gewebe gebracht wird,
**dadurch gekennzeichnet, dass**
e) der Körper ein Metall umfasst, das im Wesentlichen aus Silber mit 1,5 bis 4 % Germanium und 1 bis 2 % Indium besteht.

11. Elektrochirurgische Elektrode (40) nach Anspruch 10, wobei die aktive Fläche mit einer abgestumpften konischen Form (62) konfiguriert ist, die in einem im Allgemeinen abgeflachten distalen Ende (64) endet.

12. Elektrochirurgische Elektrode (40) nach Anspruch 10, wobei der kugelförmige Körper eine geschärfte Spitze (44) umfasst.

13. Elektrochirurgische Elektrode (24) nach Anspruch 1, in Verbindung und für eine Verwendung mit einer elektrochirurgischen Vorrichtung (20), die dazu in der Lage ist, elektrochirurgische HF-Ströme mit einer Frequenz von etwa 3,8 bis 4 MHz zu liefern.

14. Elektrochirurgische Elektrode (24) nach Anspruch 1, in Verbindung und für eine Verwendung mit einer elektrochirurgischen Vorrichtung (20), die dazu in der Lage ist, elektrochirurgische Ströme im Bereich von 2 bis 4 MHz zu liefern.

15. Verfahren zum Fertigen einer elektrochirurgischen Elektrode (24) nach Anspruch 1, das umfasst, den Körper zu fertigen durch Wickeln einer verhältnismäßig dicken Folie aus dem Plattierungsmetall (16) um einen verhältnismäßig dicken Draht oder Stab aus dem Kernmetall (14), Erhitzen bei einer erhöhten Temperatur unterhalb des Schmelz- oder Erweichungspunktes des Plattierungsmetalls (16) und des Molybdäns, um das Plattierungsmetall (16) eng an den Draht oder Stab (14) zu binden, Ziehen des verhältnismäßig dicken plattierten Drahtes oder Stabes durch eine erste Düse, was den Durchmesser um etwa 10 bis 20 % verringert, danach Tempern des gezogenen plattierten Drahtes oder Stabes, um die Verformbarkeit des Molybdäns wiederherzustellen, danach Ziehen des dünneren plattierten Drahtes oder Stabes durch eine zweite Düse, was den Durchmesser um etwa weitere 10 bis 20 % verringert, erneutes Tempern, um die Verformbarkeit des Molybdäns wiederherzustellen, und so weiter, bis der sich ergebende plattierte Draht oder Stab den kleineren Durchmesser von 0,10 bis 0,64 mm (4 bis 25 Milli-Inch) erreicht hat.

## Revendications

1. Electrode électrochirurgicale (24) comprenant :
a) un corps comprenant un métal de coeur (14) essentiellement constitué par du molybdène,
b) le corps ayant une première extrémité configurée pour être fixée à ou montée sur une pièce à main électrochirurgicale,
c) le corps ayant une deuxième extrémité active (26) opposée à la première extrémité capable de fournir des courants électrochirurgicaux au tissu lorsque la première extrémité est connectée à un appareil électrochirurgical (20),
d) ladite deuxième extrémité active (26) comprenant une surface active qui est configurée pour réaliser une action de découpe ou de coagulation lors de son activation par des courants électrochirurgicaux et lorsque la surface active est mise en contact avec le tissu, **caractérisée par** :
e) un métal de gainage (16) étroitement lié et adhérant au métal de coeur et essentiellement constitué par de l'argent avec 1,5 à 4 % de germanium et 1 à 2 d'indium.

2. Electrode électrochirurgicale (24) selon la revendication 1, dans laquelle la surface active est un point.

3. Electrode électrochirurgicale (24) selon la revendication 2, dans laquelle le corps d'électrode comprend une aiguille ayant une tige et une extrémité pointue (26), la tige ayant un diamètre d'environ 0,10 à 0,64 mm (4 à 25 millième).

4. Electrode électrochirurgicale (24) selon la revendication 3, dans laquelle le métal de gainage (16) a une épaisseur d'environ 0,025 à 0,18 mm (1 à 7 millièmes) .

5. Electrode électrochirurgicale (24) selon la revendication 4, dans laquelle l'épaisseur du gainage (16) est d'environ 5 à 15 % de l'épaisseur globale de l'électrode (24).

6. Electrode électrochirurgicale (32) selon la revendication 1, l'électrode (32) comprenant une partie généralement plate ayant le long d'une première partie avant ou latérale de sa périphérie, se projetant sur les côtés ou vers l'avant de la partie plate dans une direction opposée à la première extrémité, un bord affûté exposé (34) servant de surface active.

7. Electrode électrochirurgicale (32) selon la revendication 6, comprenant en outre un point faisant saillie (36) se projetant de manière orthogonale par rapport à la partie plate de l'électrode (32).

8. Electrode électrochirurgicale (24) selon la revendication 1, l'électrode (10) comprenant une boucle (22).

9. Electrode électrochirurgicale (24) selon la revendication 1, l'électrode (10) comprenant une pince (28).

10. Electrode électrochirurgicale (40) comprenant :
a) un corps,
b) le corps ayant une tige (42) et une première extrémité configurée pour être fixée à ou montée sur une pièce à main électrochirurgicale,
c) le corps ayant une deuxième extrémité active opposée à la première extrémité capable de fournir des courants électrochirurgicaux au tissu lorsque la première extrémité est connectée à un appareil électrochirurgical,
d) dans laquelle soit
(i) ledit corps est généralement en forme de balle et ladite deuxième extrémité active comprend une surface active qui est configurée pour réaliser une action de découpe ou de coagulation lors de son activation par des courants électrochirurgicaux et lorsque la surface active est mise en contact avec le tissu, soit
(ii) ledit corps est généralement en forme d'aiguille et ladite deuxième extrémité active comprend un point affûté qui est configuré pour réaliser une action de découpe lors de son activation par des courants électrochirurgicaux et de sa mise en contact avec le tissu,
**caractérisée en ce que** :
e) le corps comprend un métal essentiellement constitué par de l'argent avec 1,5 à 4 % de germanium et 1 à 2 % d'indium.

11. Electrode électrochirurgicale (40) selon la revendication 10, dans laquelle la surface active est configurée selon une forme conique tronquée (62) se terminant en une extrémité distale généralement plate (64).

12. Electrode électrochirurgicale (40) selon la revendication 10, dans laquelle le corps en forme de balle comprend un point affûté (44).

13. Electrode électrochirurgicale (24) selon la revendication 1, en combinaison et destinée à être utilisée avec un appareil électrochirurgical (20) capable de fournir des courants électrochirurgicaux RF à une fréquence d'environ 3,8 à 4 MHz.

14. Electrode électrochirurgicale (24) selon la revendication 1, en combinaison et destinée à être utilisée avec un appareil électrochirurgical (20) capable de fournir des courants électrochirurgicaux dans la plage de 2 à 4 MHz.

15. Procédé de fabrication d'une électrode électrochirurgicale (24) selon la revendication 1, qui comprend la fabrication du corps par enroulement d'une feuille relativement épaisse du métal de gainage (16) autour d'un fil ou d'une tige relativement épais(se) du métal de coeur (14), le chauffage à une température élevée inférieure au point de fusion ou de ramollissement du métal de gainage (16) et du molybdène pour lier étroitement le métal de gainage (16) au coeur de fil ou de tige (14), l'étirement du fil ou de la tige de gaine relativement épais(se) à travers une première filière, ce qui réduit le diamètre d'environ 10 à 20 %, puis le recuit du fil ou de la tige étiré(e) pour restaurer la ductilité du molybdène, puis l'étirement du fil ou de la tige de gaine plus fin(e) à travers une deuxième filière, ce qui réduit le diamètre d'environ 10 à 20 % supplémentaires, de nouveau le recuit pour restaurer la ductilité du molybdène, et ainsi de suite jusqu'à ce que le fil ou la tige de gaine résultant(e) ait atteint le diamètre inférieur de 0,10 à 0,64 mm (4 à 25 millièmes).
